(19) Europäisches Patentamt
European Patent Office
Office européen des brevets

(11) Veröffentlichungsnummer: **0 113 831**
**A2**

# (12) EUROPÄISCHE PATENTANMELDUNG

(21) Anmeldenummer: **83111526.6**

(22) Anmeldetag: **18.11.83**

(51) Int. Cl.³: **C 07 D 241/44**, C 07 D 215/22, C 07 D 213/64, A 01 N 47/12 // C07C103/44, C07C103/375, C07C125/065

(30) Priorität: **17.12.82 CH 7369/82**
**21.09.83 CH 5130/83**

(43) Veröffentlichungstag der Anmeldung: **25.07.84**
**Patentblatt 84/30**

(84) Benannte Vertragsstaaten: **AT BE CH DE FR GB IT LI NL**

(71) Anmelder: **F. HOFFMANN-LA ROCHE & CO. Aktiengesellschaft, CH-4002 Basel (CH)**

(72) Erfinder: **Wenger, Jean, Dr., Brunnenwiesenstrasse 1, CH-8610 Uster (CH)**
Erfinder: **Zurflüh, René, Dr., Dachslenbergstrasse 54, CH-8180 Bülach (CH)**

(74) Vertreter: **Aschert, Hubert et al, Grenzacherstrasse 124 Postfach 3255, CH-4002 Basel (CH)**

## (54) Carbaminsäureester, Verfahren zu deren Herstellung und deren Verwendung.

(57) Carbaminsäureester der Formel

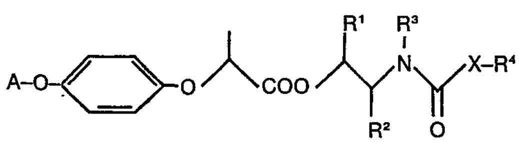

worin A eine der Gruppen (a) und (b)

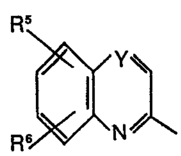

(a)

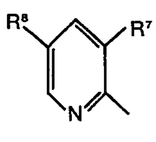

(b)

darstellt, worin $R^1$ Wasserstoff, Methyl oder Äthyl, $R^2$ Wasserstoff, Methyl oder Äthyl, $R^3$ Wasserstoff oder Alkyl, $R^4$ Alkyl oder halogensubstituiertes Alkyl, $R^5$ Halogen, Alkyl, halogensubstituiertes Methyl, Alkoxy, halogensubstituiertes Methoxy, $R^6$ Wasserstoff oder Chlor, $R^7$ Wasserstoff oder Chlor, $R^8$ Chlor, halogensubstituiertes Methyl, X Sauerstoff oder Schwefel und Y Stickstoff oder –CH= bedeuten.

Verfahren zur Herstellung dieser Verbindungen und deren Verwendung zur Unkrautbekämpfung.

EP 0 113 831 A2

F.Hoffmann-La Roche & Co. Aktiengesellschaft,Basel,Schweiz

RAN 6102/38

# Carbaminsäureester, Verfahren zu deren Herstellung und deren Verwendung

Die vorliegende Erfindung betrifft Carbaminsäureester, nämlich Verbindungen der allgemeinen Formel

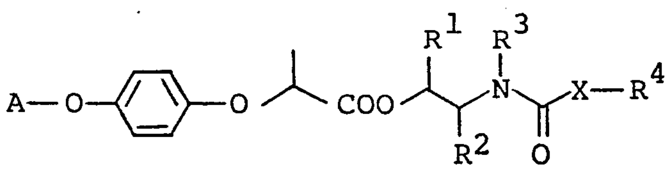

I

worin A eine der Gruppen (a) und (b)

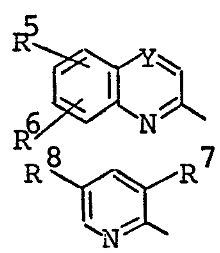

(a)

(b)

darstellt, worin $R^1$ Wasserstoff, Methyl oder Aethyl, $R^2$ Wasserstoff, Methyl oder Aethyl, $R^3$ Wasserstoff oder $C_{1-4}$-Alkyl, $R^4$ $C_{1-4}$-Alkyl oder halogensubstituiertes $C_{1-4}$-Alkyl, $R^5$ Halogen, $C_{1-4}$-Alkyl, halogensubstituiertes Methyl, $C_{1-4}$-Alkoxy, halogensubstituiertes Methoxy, $R^6$ Wasserstoff oder Chlor, $R^7$ Wasserstoff oder Chlor, $R^8$ Chlor, halogensubstituiertes Methyl,

X Sauerstoff oder Schwefel und Y Stickstoff oder -CH= bedeuten.

Die Verbindungen der Formel I besitzen herbizide Eigenschaften und eignen sich somit als Unkrautbekämpfungsmittel bzw. als Wirkstoffe von Unkrautbekämpfungsmitteln. Somit umfasst die Erfindung auch Unkrautbekämpfungsmittel, welche wenigstens eine Verbindung der Formel I als Wirkstoff enthalten, Verfahren zur Herstellung dieser Verbindungen sowie die Verwendung solcher Verbindungen bzw. Mittel zur Bekämpfung von Unkräutern.

Unter "$C_{1-4}$-Alkyl" sind sowohl geradkettige als auch verzweigte Alkylgruppen zu verstehen, wie Methyl, Aethyl, n-Propyl, Isopropyl, n-Butyl, Isobutyl und tert.Butyl.

Unter "Halogen" sind Fluor, Chlor, Brom und Jod zu verstehen, wobei Chlor bevorzugt ist. Ein bevorzugter halogensubstituierter $C_{1-4}$Alkylrest ist der 2-Chloräthylrest.

Unter "$C_{1-4}$Alkoxy" sind Alkoxygruppen zu verstehen, deren Alkylanteil der obigen Definition für $C_{1-4}$Alkyl entspricht.

Unter "halogensubstituiertes Methyl" ist insbesondere zu verstehen Trifluormethyl, Difluormethyl und Difluorchlormethyl.

Unter "halogensubstituiertes Methoxy" ist insbesondere zu verstehen Difluormethoxy und Trifluormethoxy.

Da in der Phenoxypropionylgruppierung der Verbindungen der Formel I ein asymmetrisches C-Atom vorhanden ist und da gegebenenfalls weitere asymmetrische Kohlenstoffatome in der Esterkomponente vorhanden sein können,

können die Verbindungen der Formel I in optisch aktiver Form auftreten. Die Formel I soll demnach die möglichen optischen Isomeren sowie die Racemate umfassen. Dem Asymmetriezentrum in der α-Stellung zum Carbonylsauerstoff kommt eine besondere Bedeutung zu. Die D-Form der Verbindungen der Formel I ist im Hinblick auf ihre besondere Wirkungsqualität bevorzugt.

In der obigen Formel I sind $R^2$ und $R^3$ vorzugsweise Wasserstoff, $R^4$ bedeutet vorzugsweise Methyl oder Aethyl, $R^5$ bedeutet vorzugsweise Fluor, Chlor oder Trifluormethyl, $R^6$ bedeutet vorzugsweise Wasserstoff, $R^8$ bedeutet vorzugsweise Difluorchlormethyl oder Trifluormethyl und X ist vorzugsweise Sauerstoff.

Besonders bevorzugt sind die Verbindungen der Formel I, worin A eine Gruppe der Formel (a) darstellt, und zwar insbesondere jene Verbindungen, worin Y Stickstoff bedeutet, das heisst die Chinoxalinverbindungen der Formel I (a). Bei diesen Chinoxalinverbindungen ist insbesondere die D-Form bevorzugt.

Besonders bevorzugte einzelne Verbindungen der Formel I sind:

Der 2-[[D-2-[p-[(6-Chlor-2-chinoxalinyl)oxy]-phenoxy]propionyl]oxy]äthylcarbaminsäure-methylester,
der 2-[[D-2-[p-[(6-Chlor-2-chinoxalinyl)oxy]phenoxy]-propionyl]oxy]propylcarbaminsäure-methylester,
der 2-[[D-2-[p-[[5-(Trifluormethyl)-2-pyridyl]-oxy]phenoxy]propionyl]oxy]äthylcarbaminsäure-methylester,
der 2-[[D-2-[p-[[5-Trifluormethyl)-2-pyridyl]oxy]-phenoxy]propionyl]oxy]äthylcarbaminsäure-äthylester,
der 2-[[D-2[p-[[5-Trifluormethyl-2-pyridyl]oxy]-phenoxy]propionyl]oxy]propylcarbaminsäure-methylester.

Weitere bevorzugte Verbindungen der Formel I sind:

der 2-[[D-2-[p-[(6-Chlor-2-chinoxalinyl)oxy]phenoxy]-propionyl]oxy]äthylcarbaminsäure-äthylester,

der 2-[[D-2-[p-[(6-Chlor-2-chinoxalinyl)oxy]phenoxy]-propionyl]oxy]-N-methyl-äthylcarbaminsäure-methylester,

der 2-[[D-2-[p-[(6-Chlor-2-chinoxalinyl)oxy]phenoxy]-propionyl]oxy]1-(äthyl)äthylcarbaminsäure-äthylester,

der 2-[[D-2-[p-[(6-Chlor-2-chinoxalinyl)oxy]phenoxy]-propionyl]oxy]äthylcarbaminsäure-isobutylester,

der 2-[[2-[p-[(6-Chlor-2-chinoxalinyl)oxy]phenoxy]-propionyl]oxy]äthylcarbaminsäure-methylester,

der 2-[[D-2-[p-[(6-Fluor-2-chinoxalinyl)oxy]phenoxy]-propionyl]oxy]äthylcarbaminsäure-äthylester und

der 2-[[D-2-[p-[(6-Fluor-2-chinoxalinyl)oxy]phenoxy]-propionyl]oxy]propylcarbaminsäure-methylester.

Weitere interessante Vertreter der Verbindungen der Formel I sind:

Der 2-[[D-2-[p-[(6-Chlor-2-chinoxalinyl)oxy]phenoxy]-propionyl]oxy-N-methyl-äthylcarbaminsäure-thioäthylester,

der 2-[[D-2-[p-[(6-Methyl-2-chinoxalinyl)oxy]phenoxy]-propionyl]oxy]äthylcarbaminsäure-methylester,

der 2-[[D-2-[p-[(6-Trifluormethyl-2-chinoxalinyl)oxy]-phenoxy]propionyl]oxy]äthylcarbaminsäure-methylester,

der 2-[[D-2-[p-[(6,7-Dichlor-2-chinoxalinyl)oxy]-phenoxy]propionyl]oxy]äthylcarbaminsäure-methylester,

der 2-[[D-2-[p-[(6-Chlor-2-chinoxalinyl)oxy]-phenoxy]propionyl]oxy]N-methyl-äthylcarbaminsäure-thio-äthylester,

der 2-[[D-2-[p-[(6-Fluor-2-chinoxalinyl)oxy]phenoxy]-propionyl]oxy]äthylcarbaminsäure-methylester,

der 2-[[2-[p-[(6-Chlor-2-chinolinyl)oxy]phenoxy]-propionyl]oxy]äthylcarbaminsäure-methylester,

der 2-[[2-[p-[(6-Chlor-2-chinolinyl)oxy]phenoxy]-propionyl]oxy]propylcarbaminsäure-methylester,

der 2-[[D-2-[p-[(6-Chlor-2-chinolinyl)oxy]phenoxy]-propionyl]oxy]äthylcarbaminsäure-methylester,

der 2-[[D-2-[p-[(6-Chlor-2-chinolinyl)oxy]phenoxy]-

propionyl]oxy]propylcarbaminsäure-methylester,

der 2-[[D-2-[p-[(6-Brom-2-chinolinyl)oxy]phenoxy]-propionyl]oxy]äthylcarbaminsäure-äthylester,

der 2-[[D-2-[p-[(6-Fluor-2-chinolinyl)oxy]phenoxy]-propionyl]oxy]äthylcarbaminsäure-äthylester,

der 2-[[D-2-[p-[(6-Fluor-2-chinolinyl)oxy]phenoxy]-propionyl]oxy]äthylcarbaminsäure-methylester und

der 2-[[D-2-[p-[(6-Fluor-2-chinolinyl)oxy]phenoxy]-propionyl]oxy]propylcarbaminsäure-methylester.

Das erfindungsgemässe Verfahren zur Herstellung der Verbindungen der Formel I ist dadurch gekennzeichnet, dass man

a) eine substituierte Phenoxy-propionsäure der allgemeinen Formel

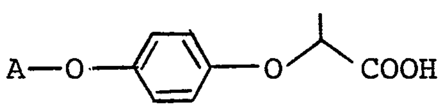

II

worin A die oben angegebene Bedeutung besitzt, oder ein reaktionsfähiges Derivat davon, mit einer Verbindung der allgemeinen Formel

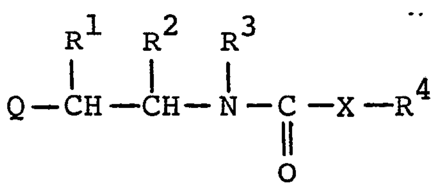

III

worin $R^1$, $R^2$, $R^3$, $R^4$ und X die oben angegebenen Bedeutungen besitzen und Q eine Abgangsgruppe darstellt,

umsetzt, oder

b) eine Verbindung der allgemeinen Formel

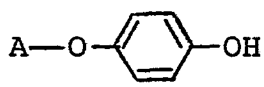

IV

worin A die oben angegebene Bedeutung besitzt,

oder ein Alkalimetallsalz davon, mit einer Verbindung der allgemeinen Formel

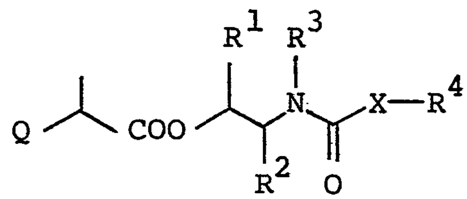

V

worin Q eine Abgangsgruppe darstellt und $R^1$, $R^2$, $R^3$, $R^4$ und X die oben angegebenen Bedeutungen besitzen,

erforderlichenfalls in Gegenwart einer Base, umsetzt, oder

c) eine Verbindung der allgemeinen Formel

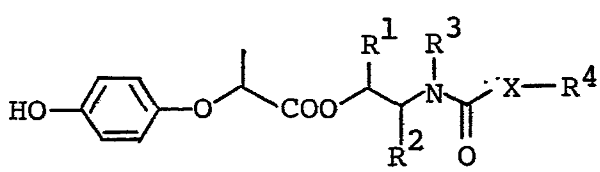

VI

worin $R^1$, $R^2$, $R^3$, $R^4$ und X die oben angegebenen Bedeutungen besitzen,

oder ein Alkalimetallsalz davon, mit einer Verbindung der allgemeinen Formel

AZ VII

worin A eine der obigen Gruppen (a) und (b) darstellt und Z Halogen, insbesondere Chlor oder Fluor, bedeutet,

umsetzt.

Der Ausdruck "Abgangsgruppe" (Q) bedeutet vorzugsweise Chlor, Brom, Jod, Mesyloxy oder Tosyloxy.

Gemäss der Verfahrensvariante a) wird eine Verbindung der Formel II oder ein reaktionsfähiges Derivat davon mit einer Verbindung der Formel III verestert. Als reaktionsfähiges Derivat wird insbesondere ein Salz, beispielsweise ein Alkalimetallsalz, z.B. das Natrium-, Kalium- oder Lithiumsalz, ein Erdalkalimetallsalz, z.B. das Magnesium-, Kalzium- oder Bariumsalz, ein Salz einer organischen Base, z.B. ein Mono-, Di- oder Trialkylammoniumsalz oder ein Pyridiniumsalz, oder das Ammoniumsalz verwendet.

Die Veresterung der Säure der Formel II, vorzugsweise in Form eines Salzes, mit der Verbindung der Formel III wird zweckmässig in einem inerten Lösungsmittel und bei Temperaturen von etwa -20°C bis 100°C durchgeführt. Ein besonders bevorzugter Temperaturbereich ist derjenige zwischen 0°C und 40°C. Als Lösungsmittel kommen vorzugsweise inerte organische Lösungsmittel in Frage. Hierzu gehören vorzugsweise Aether, z.B. der Diäthyläther und Tetrahydrofuran, chlorierte Kohlenwasserstoffe, z.B. Dichlormethan und Chloroform, und Dimethylformamid.

Bei Verwendung der freien Säure der Formel II wird die Umsetzung zweckmässigerweise in Gegenwart einer Base bzw. eines Säureakzeptors durchgeführt. Man kann hierfür alle üblicherweise verwendbaren anorganischen und organischen säurebindenden Mittel benutzen, vorzugsweise Alkalimetall- und Erdalkalimetallcarbonate- und -bicarbonate, und tertiäre Amine, z.B. Triäthylamin, Dimethylanilin und Pyridin.

Als reaktionsfähiges Derivat der Formel II kann auch ein Halogenid, Imidazolid oder Anhydrid der Säure der Formel II verwendet werden, wobei man dann als Reaktionspartner eine Verbindung der Formel III verwendet, worin Q für Hydroxy steht.

Auch die freie Säure der Formel II kann mit einer Verbindung der Formel III umgesetzt werden, worin Q Hydroxy bedeutet. Diese Umsetzung erfolgt zweckmässigerweise in Gegenwart eines sauren Katalysators bzw. eines kondensierenden Mittels, wie beispielsweise Schwefelsäure, Salzsäure, p-Toluolsulfonsäure, Dicyclohexylcarbodiimid oder Carbonyldiimidazol.

Die Verfahrensvariante b) oder c) kann zweckmässigerweise derart durchgeführt werden, dass man das Phenol der Formel IV bzw. VI in Form eines Alkalimetallsalzes, beispielsweise des Natriumsalzes, mit einem Halogenid der Formel V bzw. VII umsetzt. Dies erfolgt zweckmässigerweise in einem aprotischen Lösungsmittel, z.B. in Dimethylformamid oder Dimethylsulfoxyd, oder in Pyridin, bei Temperaturen zwischen etwa 50°C und 150°C, vorzugsweise zwischen 70°C und 110°C.

Die Isolierung und Reinigung der so erhaltenen Verbindungen der Formel I kann nach an sich bekannten Methoden erfolgen.

Wenn bei der Herstellung der Verbindungen der Formel I keine gezielte Synthese zur Gewinnung reiner optischer Isomerer angewandt wurde (Verwendung von optisch reinem Ausgangsmaterial), fällt das Produkt normalerweise als Racemat an. In diesem Fall kann das Racemat nach an sich bekannten Methoden in die Isomeren aufgetrennt werden. Unter Verwendung optisch aktiver Ausgangsmaterialien erhält man entsprechend optisch aktive Produkte.

Die Ausgangsmaterialien der Formeln II, IV und VII sowie reaktionsfähige Derivate der Säuren der Formel II und Alkalimetallsalze der Phenole der Formel IV sind entweder bekannt oder können nach an sich bekannten Methoden hergestellt werden.

Die Ausgangsmaterialien der Formeln III und V können in an sich bekannter Weise, z.B. nach folgendem Reaktionsschema, hergestellt werden. Im Reaktionsschema besitzen die Symbole $R^1$, $R^2$, $R^3$, $R^4$, X und Q die oben angegebene Bedeutungen und bedeutet Hal ein Halogenatom, insbesondere Chlor oder Brom.

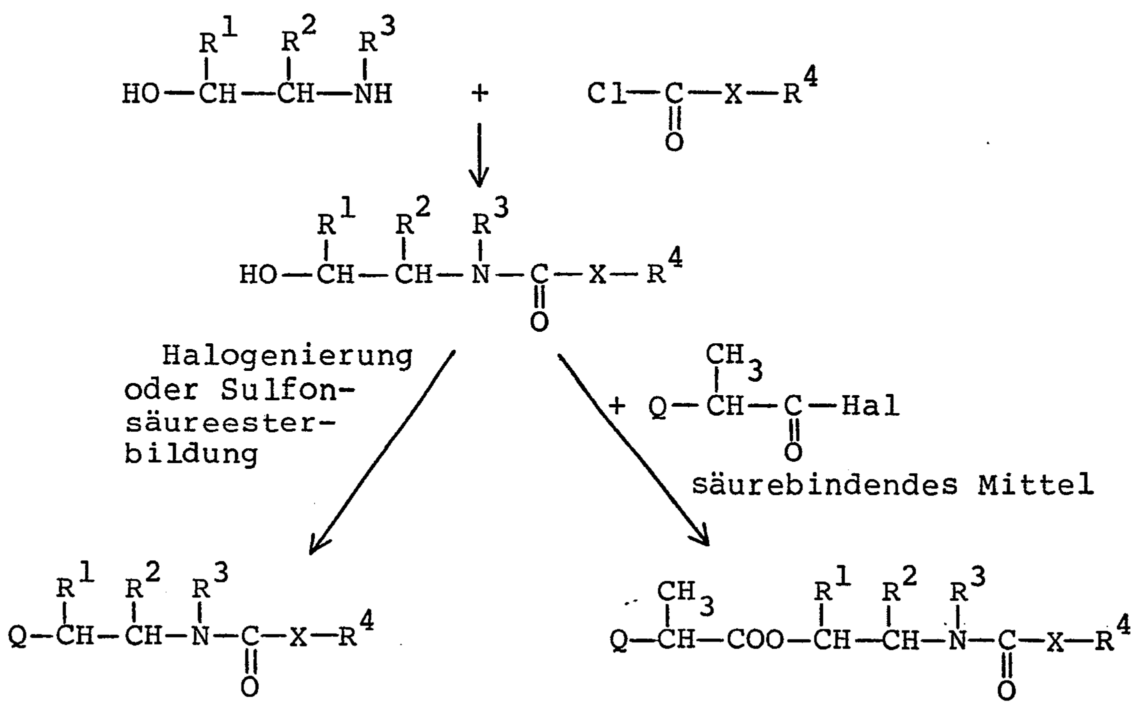


III V

Die Ausgangsmaterialien der Formel VI sind neu. Sie können dadurch hergestellt werden, dass man eine Verbindung der allgemeinen Formel

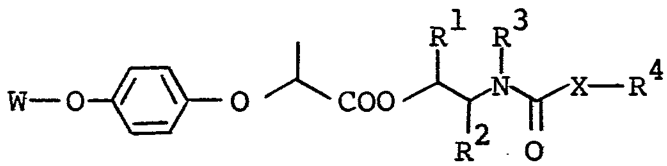

VIII

worin $R^1$, $R^2$, $R^3$, $R^4$ und X die oben angegebenen Bedeutungen besitzen und W eine leicht abspaltbare Gruppe, insbesondere Benzyl, bedeutet,

unter Abspaltung von W in eine Verbindung der Formel VI überführt.

Die Verbindungen der Formel VIII können nach folgendem Reaktionsschema hergestellt werden, wobei die Symbole $R^1$, $R^2$, $R^3$, $R^4$, X, Q und W die oben angegebenen Bedeutungen besitzen.

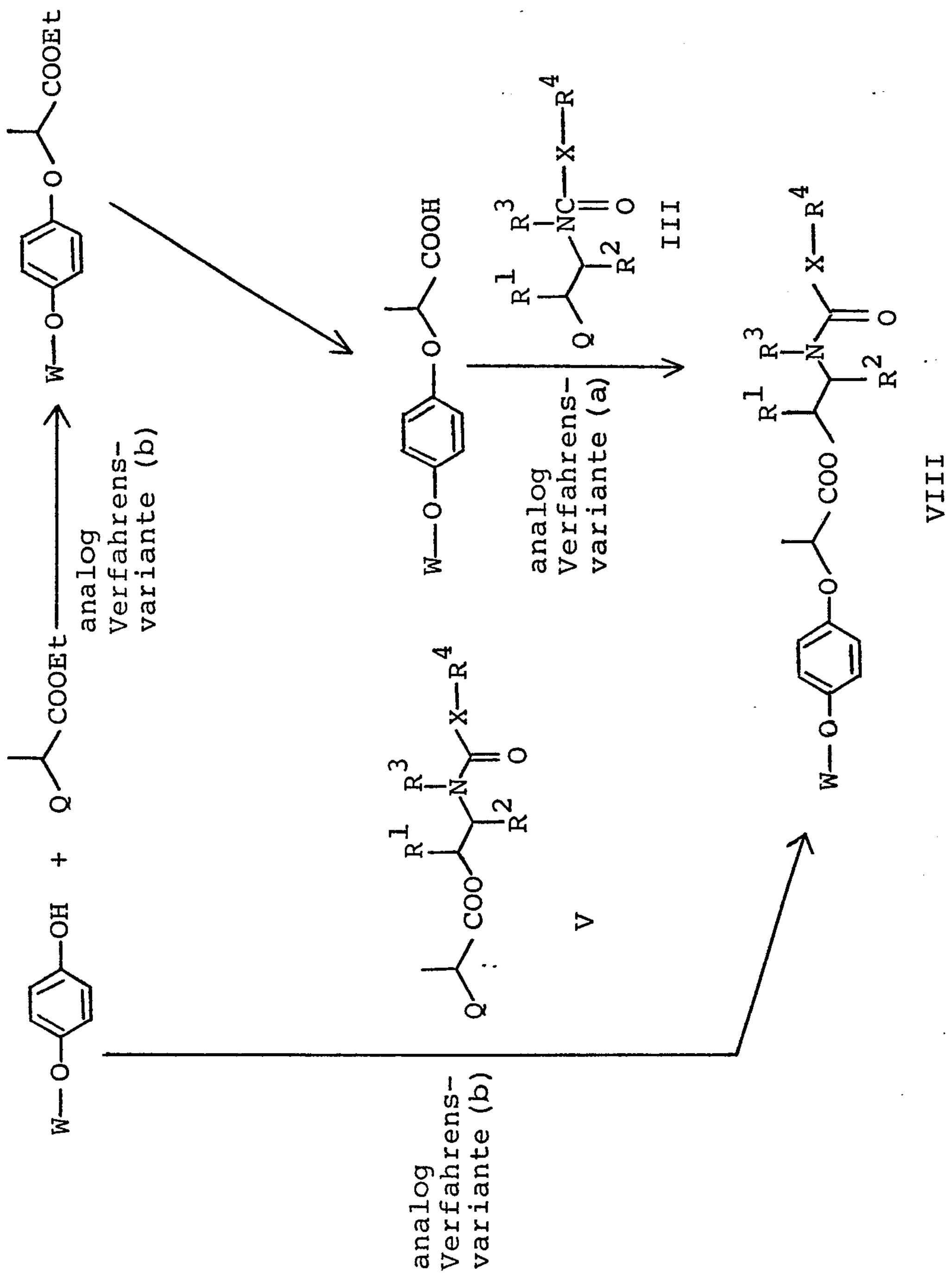
analog Verfahrensvariante (b)
analog Verfahrensvariante (a)
analog Verfahrensvariante (b)

V

III

VIII

Für die für die Herstellung der Ausgangsmaterialien der Formel VII, worin A die Gruppe (a) bedeutet, worin Y Stickstoff darstellt, d.h. für die für die Herstellung der Ausgangsmaterialien der Formel

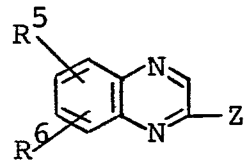

VIIa

worin Z ein Halogenatom, insbesondere Chlor, bedeutet und $R^5$ und $R^6$ die obigen Bedeutungen haben,

benötigten 2-Hydroxychinoxaline der Formel

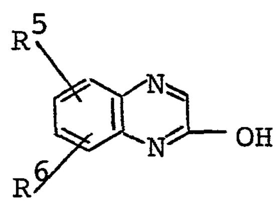

IX

worin $R^5$ und $R^6$ die oben angegebene Bedeutung besitzen,

wurde im Rahmen der vorliegenden Erfindung ein neues Verfahren gefunden, welches ebenfalls Gegenstand der vorliegenden Erfindung bildet. Dieses Verfahren ist dadurch gekennzeichnet, dass man eine Verbindung der Formel

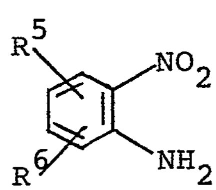

X

mit einer den Rest der Formel

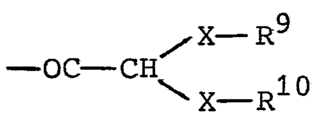

XI

abgebenden Verbindung zu einem Amid der Formel

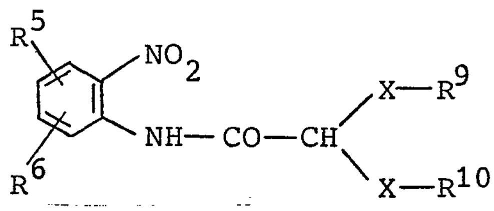

XII

umsetzt, und dass man in beliebiger Reihenfolge die Nitrogruppe in der Verbindung der Formel XII zur Aminogruppe reduziert und die Gruppen $-X-R^9$ und $-X-R^{10}$ abspaltet, wodurch sich unter Ringschluss die Verbindung der Formel IX bildet.

In den obigen Formeln X, XI und XII haben die Substituenten $R^5$ und $R^6$ die weiter oben angegebenen Bedeutungen, X bedeutet Sauerstoff oder Schwefel und $R^9$ und $R^{10}$ bedeuten unabhängig voneinander $C_1$-$C_6$Alkyl, welches gegebenenfalls mit Halogen, niederem Alkoxy oder niederem Alkylthio substituiert ist, ferner $C_2$-$C_4$Acyl, z.B. Acetyl oder Propionyl. $R^9$ und $R^{10}$ zusammen können auch eine Alkylengruppe mit 2 oder 3 Kohlenstoffstomen bedeuten.

Als den Rest der Formel XI abgebende Verbindung kann ein Ester, beispielsweise ein nieder Alkylester, z.B. der Aethylester verwendet werden, oder ein Halogenid, z.B. das entsprechende Säurechlorid oder ein anderes reaktionsfähiges Derivat.

Die Umsetzung einer Verbindung der Formel X mit einer den Rest der Formel XI abgebenden Verbindung, beispielsweise dem Aethylester, erfolgt zweckmässig nach der in Tetrahedron Letters 1971, p. 321-22 beschriebenen Methode, nämlich mittels eines Alkalimetallhydrids, z.B. Natriumhydrid, in Dimethylsulfoxyd, Hexamethylphosphoramid, N-Methyl-2-pyrrolidon, Tetramethylharnstoff, Dimethylformamid oder N,N-Dimethylacetamid, und zwar bei Temperaturen zwischen Raumtemperatur und dem Siedepunkt des Reaktionsgemisches. Zweckmässig werden hierbei äquimolare Mengen von Amin, Ester und Hydrid verwendet.

In der so erhaltenen Verbindung der Formel XII wird hierauf in beliebiger Reihenfolge die Nitrogruppe zur Aminogruppe reduziert und die Abspaltung der Gruppen $-X-R^9$ und $-X-R^{10}$ vorgenommen. Die Reduktion der Nitrogruppe erfolgt zweckmässig mit Wasserstoff in Gegenwart eines Katalysators, wie Raney-Nickel, Palladium oder dergleichen, oder mit Hydrazin, gegebenenfalls in Form des Monohydrats, in Gegenwart von Ferrichlorid oder eines der obigen Katalysatoren. Diese Reduktion kann auch mittels eines Alkalimetallsulfids, wie Natriumsulfid, oder Ammonsulfid oder einem Alkalimetalldithionit, z.B. mit Natriumdithionit erfolgen. Zweckmässig erfolgt die Reduktion in einem Temperaturbereich zwischen etwa 0 und 30°C, vorzugsweise etwa bei Raumtemperatur.

Die nach Reduktion der Verbindung der Formel XII und Abspaltung der Gruppen $-X-R^9$ und $-X-R^{10}$ erhaltene Aminoverbindung cyklisiert zur Verbindung der Formel IX. Für die Abspaltung wird zweckmässig ein saurer Katalysator verwendet, beispielsweise eine Lewis-Säure, z.B. ein saurer Ionenaustauscher, wie beispielsweise Amberlyst 15 oder Amberlit 120. Zweckmässig erfolgt diese Abspaltung und der Ringschluss in einem inerten, mit Wasser mischbaren Lösungsmittel, z.B. Alkohol, Aceton oder dgl. Die Ringschlussreaktion kann bei Temperaturen zwischen Raumtemperatur und der Rückflusstemperatur des Reaktionsgemisches, vorzugsweise bei letzterer, erfolgen.

Die so erhaltene Verbindung der Formel IX kann durch Halogenierung in an sich bekannter Weise in die Chinoxalinverbindung der Formel VII übergeführt werden.

Die Verbindungen der Formel I besitzen herbizide Eigenschaften und eignen sich zur Bekämpfung von Gräsern, insbesondere Echinochloa crus-galli, Setaria faberii, Agropyron repens, Sorghum bicolor, Alopecurus myosuroides, Avena fatua in Soya-, Zuckerrüben- und Baumwollkulturen. Besonders gut eignen sich die erfindungsgemässen Verbindungen zur Bekämpfung von Gräsern in Soyakulturen.

Im allgemeinen genügt eine Konzentration von 0,01 bis 0,5 kg Wirkstoff der Formel I/ha, vorzugsweise 0,05 bis 0,2 kg/ha Wirkstoff der Formel I/ha, um den gewünschten herbiziden Effekt zu erzielen.

Die Verbindungen der Formel I sind sowohl Vorauflauf-Herbizide, als auch Nachauflauf-Herbizide, wobei ihre Anwendung als Nachauflauf-Herbizide bevorzugt ist.

Das erfindungsgemässe Unkrautbekämpfungsmittel ist dadurch gekennzeichnet, dass es eine wirksame Menge mindestens einer Verbindung der Formel I, wie oben definiert, sowie Formulierungshilfsstoffe enthält. Das Mittel enthält zweckmässigerweise zumindest einen der folgenden Formulierungshilfsstoffe: feste Trägerstoffe; Lösungs- bzw. Dispersionsmittel; Tenside (Netz- und Emulgiermittel); Dispergatoren (ohne Tensidwirkung); und Stabilisatoren. Unter Verwendung solcher und anderer Hilfsstoffe können die Verbindungen der Formel I, also die herbiziden Wirkstoffe, in die üblichen Formulierungen übergeführt werden, wie Stäube, Pulver, Granulate, Lösungen, Emulsionen, Suspensionen, emulgierbare Konzentrate, Pasten und dergleichen.

Die Verbindungen der Formel I sind im allgemeinen wasserunlöslich und können nach den für wasserunlösliche Verbindungen üblichen Methoden unter Verwendung der diesbezüglichen Formulierungshilfsstoffe konfektioniert werden. Die Herstellung der Mittel kann in an sich bekannter Weise durchgeführt werden, z.B. durch Vermischen des jeweiligen Wirkstoffes mit festen Trägerstoffen, durch Auflösen oder Suspendieren in geeigneten Lösungs- bzw. Dispersionsmitteln, eventuell unter Verwendung von Tensiden als Netz- oder Emulgiermitteln und/oder von Dispergatoren, durch Verdünnen bereits vorbereiteter emulgierbarer Konzentrate mit Lösungs- bzw. Dispersionsmitteln usw.

Als feste Trägerstoffe kommen im wesentlichen in Frage: natürliche Mineralstoffe, wie Kreide, Dolomit, Kalkstein, Tonerden und Kieselsäure und deren Salze (beispielsweise Kieselgur, Kaolin, Bentonit, Talkum, Attapulgit und Montmorillonit); synthetische Mineralstoffe, wie hochdisperse Kieselsäure, Aluminiumoxid und Silikate; organische Stoffe, wie Cellulose, Stärke, Harnstoff und Kunstharze; und Düngemittel, wie Phosphate und Nitrate, wobei solche Trägerstoffe z.B. als Pulver oder als Granulate vorliegen können.

Als Lösungs- bzw. Dispersionsmittel kommen im wesentlichen in Frage: Aromaten, wie Benzol, Toluol, Xylole und Alkylnaphthaline; chlorierte Aromaten und chlorierte aliphatische Kohlenwasserstoffe, wie Chlorbenzole, Chloräthylene und Methylenchlorid; aliphatische Kohlenwasserstoffe, wie Cyclohexan und Paraffine, z.B. Erdölfraktionen; Alkohole, wie Butanol und Glykol, sowie deren Aether und Ester; Ketone, wie Aceton, Methyläthylketon, Methylisobutylketon und Cyclohexanon; und stark polare Lösungs- bzw. Dispersionsmittel, wie Dimethylformamid, N-Methylpyrrolidon und Dimethylsulfoxid, wobei solche Lösungsmittel vorzugsweise Flammpunkte von mindestens 30°C und Siedepunkt von mindestens 50°C aufweisen, und Wasser. Unter den Lösungs- bzw. Dispersionsmitteln kommen auch in Frage sogenannte verflüssigte gasförmige Streckmittel oder Trägerstoffe, die solche Produkte sind, welche bei Raumtemperatur und unter Normaldruck gasförmig sind. Beispiele solcher Produkte sind insbesondere Aerosol-Treibgase, wie Halogenkohlenwasserstoffe, z.B. Dichlordifluormethan. Liegt das erfindungsgemässe Unkrautbekämpfungsmittel in Form einer Druckgaspackung vor, so wird zweckmässigerweise zusätzlich zum Treibgas ein Lösungsmittel verwendet.

Die Tenside (Netz- und Emulgiermittel) können nichtionische Verbindungen sein, wie Kondensationsprodukte von Fettsäuren, Fettalkoholen oder fettsubstituierten Phenolen mit Aethylenoxid; Fettsäureester und -äther von Zuckern

oder mehrwertigen Alkoholen; die Produkte, die aus Zuckern oder mehrwertigen Alkoholen durch Kondensation mit Aethylenoxid erhalten werden; Blockpolymere von Aethylenoxid und Propylenoxid; oder Alkyldimethylaminoxide.

Die Tenside können auch anionische Verbindungen darstellen, wie Seifen; Fettsulfatester, z.B. Dodecylnatriumsulfat, Octadecylnatriumsulfat und Cetylnatriumsulfat; Alkylsulfonate, Arylsulfonate und fettaromatische Sulfonate, wie Alkylbenzolsulfonate, z.B. Calcium-dodecylbenzol-sulfonat, und Butylnaphthalinsulfonate; und komplexere Fettsulfonate, z.B. die Amidkondensationsprodukte von Oelsäure und N-Methyltaurin und das Natriumsulfonat von Dioctylsuccinat.

Die Tenside können schliesslich kationische Verbindungen sein, wie Alkyldimethylbenzylammoniumchloride, Dialkyldimethylammoniumchloride, Alkyltrimethylammoniumchloride und äthoxylierte quaternäre Ammoniumchloride.

Als Dispergatoren (ohne Tensidwirkung) kommen im wesentlichen in Frage: Lignin, Natrium- und Ammoniumsalze von Ligninsulfonsäuren, Natriumsalze von Maleinsäurenanhydrid-Diisobutylen-Copolymeren, Natrium- und Ammoniumsalze von sulfonierten Polykondensationsprodukten aus Naphthalin und Formaldehyd, und Sulfitablaugen.

Als Dispergatoren, die sich insbesondere als Verdickungs- bzw. Antiabsetzmittel eignen, können z.B. Methylcellulose, Carboxymethylcellulose, Hydroxyäthylcellulose, Polyvinylalkohol, Alginate, Caseinate und Blutalbumin eingesetzt werden.

Beispiele von geeigneten Stabilisatoren sind säurebindende Mittel z.B. Epichlorhydrin, Phenylglycidäther und Soyaepoxide; Antioxidantien z.B. Gallussäureester und Butylhydroxytoluol; UV-Absorber, z.B. substituierte Benzophenone, Diphenylacrylonitrilsäureester und Zimtsäure-

ester; und Deaktivatoren, z.B. Salze der Aethylendiaminotetraessigsäure und Polyglykole.

Die erfindungsgemässen Unkrautbekämpfungsmittel können zusätzlich zu den Verbindungen der Formel I Synergisten und andere Wirkstoffe, z.B. Insektizide, Akarizide, Bakterizide, anderweitige Herbizide, Fungizide, Pflanzenwachstumsregulatoren und Düngemittel, enthalten. Solche Kombinationsmittel eignen sich zur Verstärkung der Aktivität bzw. zur Verbreiterung des Wirkungsspektrums.

Die erfindungsgemässen Unkrautbekämpfungsmittel enthalten im allgemeinen zwischen 0,1 und 99 Gewichtsprozent, vorzugsweise zwischen 5 und 75 Gewichtsprozent einer bzw. mehrerer Verbindungen der Formel I als Wirkstoff(e). Sie können z.B. in einer Form vorliegen, die sich für die Lagerung und den Transport eignet. In solchen Formulierungen, z.B. emulgierbaren Konzentraten, ist die Wirkstoffkonzentration normalerweise im höheren Bereich, vorzugsweise zwischen 10 und 75 Gewichtsprozent, insbesondere zwischen 25 und 50 Gewichtsprozent. Diese Formulierungen können dann, z.B. mit gleichen oder verschiedenen inerten Stoffen, bis zu Wirkstoffkonzentrationen verdünnt werden, die sich für den praktischen Gebrauch eignen, also vorzugsweise ca. 0,1 bis 10 Gewichtsprozent, insbesondere ca. 1 bis 5 Gewichtsprozent. Die Wirkstoffkonzentrationen können jedoch auch kleiner oder grösser sein.

Wie oben erwähnt, kann die Herstellung der erfindungsgemässen Unkrautbekämpfungsmittel in an sich bekannter Weise durchgeführt werden.

Zur Herstellung pulverförmiger Präparate kann der Wirkstoff, d.h. mindestens eine Verbindung der Formel I, mit festem Trägerstoff vermischt werden, z.B. durch Zusammenmahlen; oder man kann den festen Trägerstoff mit einer Lösung oder Suspension des Wirkstoffes imprägnieren und dann die Lösungs- bzw. Dispersionsmittel durch Abdunsten, Erhitzen oder Absaugen unter vermindertem Druck entfernen. Durch Zusatz von Tensiden bzw. Dispergatoren kann man solche pulverförmige Mittel mit Wasser leicht benetzbar machen, so dass sie in wässrige Suspensionen, die sich z.B. als Spritzmittel eignen, übergeführt werden können.

Die Verbindung der Formel I kann auch mit einem Tensid und einem festen Trägerstoff zur Bildung eines netzbaren Pulvers vermischt werden, welches in Wasser dispergierbar ist, oder sie kann mit einem festen vorgranulierten Trägerstoff zur Bildung eines granulatförmigen Produktes vermischt werden.

Wenn gewünscht, kann die Verbindung der Formel I in einem mit Wasser nicht mischbaren Lösungsmittel, wie beispielsweise einem hochsiedenden Kohlenwasserstoff, gelöst werden, das zweckmässigerweise gelöste Emulgiermittel enthält, so dass die Lösung bei Zugabe zu Wasser selbstemulgierend wirkt. Andernfalls kann der Wirkstoff mit einem Emulgiermittel vermischt und das Gemisch dann mit Wasser auf die gewünschte Konzentration verdünnt werden. Zudem kann der Wirkstoff in einem Lösungsmittel gelöst und danach mit einem Emulgiermittel gemischt werden. Ein solches Gemisch kann ebenfalls mit Wasser auf die gewünschte Konzentration verdünnt werden. Auf diese Weise erhält man emulgierbare Konzentrate bzw. gebrauchsfertige Emulsionen.

Die Verwendung der erfindungsgemässen Unkrautbekämpfungsmittel kann nach üblichen Applikationsmethoden, wie Spritzen, Sprühen, Stäuben, Giessen oder Streuen, erfolgen. Das erfindungsgemässe Verfahren zur Bekämpfung von Unkräutern ist dadurch gekennzeichnet, dass man das gegen Unkräuter zu schützende Gut und/oder die Unkräuter mit einer erfindungsgemässen Verbindung bzw. einem erfindungsgemässen Unkrautbekämpfungsmittel behandelt.

Die nachfolgenden Beispiele dienen zur näheren Erläuterung der Erfindung.

## I. Herstellung der Wirkstoffe der Formel I

### Beispiel 1

56 g D-2-[p-[(6-Chlor-2-chinoxalinyl)oxy]phenoxy]-propionsäure und 77,4 ml Thionylchlorid werden zusammengegeben und 1,5 Stunden am Rückfluss erhitzt. Dann wird das Thionylchlorid unter vermindertem Druck abdestilliert und der Rückstand am Hochvakuum getrocknet. Zu dem das gebildete Säurechlorid enthaltenden Rückstand gibt man 14 g Pyridin und 100 ml Methylenchlorid, dann tropft man bei 5-15°C 23,6 g 2-Hydroxypropylcarbaminsäure-methylester, gelöst in 50 ml Methylenchlorid, zu und lässt 12 Stunden bei Raumtemperatur reagieren. Das Reaktionsgemisch wird auf Wasser gegossen und mit Aether extrahiert. Die Extrakte werden mit 2N-Natronlauge, 2N-Salzsäure, wieder mit Wasser und mit gesättigter Kochsalzlösung gewaschen, über Natriumsulfat getrocknet und am Rotationsverdampfer eingeengt. Durch Chromatographie an Kieselgel mit Hexan/Aether erhält man reinen 2-[[D-2-[p-[(6-Chlor-2-chinoxalinyl)oxy]phenoxy]propionyl]oxy]propylcarbaminsäure-methylester; Smp. 94-96°C, $[\alpha]_D^{22}$ +31,03° (c = 0,93% in $CHCl_3$).

In analoger Weise erhält man aus

a) D-2-[p-[(6-Chlor-2-chinoxalinyl)oxy]phenoxy]propionsäure und

- 2-Hydroxyäthylcarbaminsäure-methylester den 2-[[D-2-[p-[(6-Chlor-2-chinoxalinyl)]oxy]phenoxy]propionyl]oxy]-äthylcarbaminsäure-methylester; Smp. 91-94°C, $[\alpha]_D^{22}$ +28,84° (c = 0,99% in $CHCl_3$);

- 2-Hydroxyäthylcarbaminsäure-äthylester den 2-[[D-2-[p-[(6-Chlor-2-chinoxalinyl)oxy]phenoxy]propionyl]oxy]-äthylcarbaminsäure-äthylester; Smp. 96-98°C, $[\alpha]_D^{22}$ +34,96° (c = 0,39 in $CHCl_3$);

- N-(2-Hydroxyäthyl)-N-methyl-thiolcarbaminsäure-äthylester den 2-[[D-2-[p-[(6-Chlor-2-chinoxalinyl)oxy]-phenoxy]propionyl]oxy]N-methyl-äthylcarbaminsäure-thioäthyl-ester; $[\alpha]_D^{22}$ +25,43° (c = 1,33% in $CHCl_3$), $n_D^{20}$ = 1,5998;

- N-(2-Hydroxyäthyl)-N-methylcarbaminsäure-methylester den 2-[[D-2-[p-[(6-Chlor-2-chinoxalinyl)oxy]phenoxy]-propionyl]oxy]N-methyl-äthylcarbaminsäure-methylester, $n_D^{20}$ = 1,5835; $[\alpha]_D^{22}$ + 31,17° (c = o,75% in $CHCl_3$);

- 1-Aethyl-2-hydroxyäthylcarbaminsäure-äthylester den 2-[[D-2-[(p-[(6-Chlor-2-chinoxalinyl)oxy]phenoxy]propionyl]-oxy]1-(äthyl)äthylcarbaminsäure-äthylester; Smp. 71-75°C, $[\alpha]_D^{22}$ +30,41° (c = 0,91% in $CHCl_3$);

- 2-Hydroxyäthylcarbaminsäure-isobutylester den 2-[[D-2-[p-[(6-Chlor-2-chinoxalinyl)oxy]phenoxy]propionyl]-oxy]äthylcarbaminsäure-isobutylester; Smp. 75-78°C, $[\alpha]_D^{22}$ +31,77° (c = 0,99% in $CHCl_3$);

b) D-2-[p-[(6-Fluor-2-chinoxalinyl)oxy]phenoxy]propionsäure und 2-Hydroxyäthylcarbaminsäure-methylester den 2-[[D-2-[p-[(6-Fluor-2-chinoxalinyl)oxy]phenoxy]propionyl]-oxy]äthylcarbaminsäure-methylester; Smp. 103-106°C, $[\alpha]_D^{22}$ +33,08° (c = 1,10% in $CHCl_3$);

c) 2-[p-[(6-Chlor-2-chinoxalinyl)oxy]phenoxy]-propionsäure und

- 2-Hydroxyäthylcarbaminsäure-methylester den 2-[[2-[p-[(6-Chlor-2-chinoxalinyl)oxy]phenoxy]propionyl]oxy]-äthylcarbaminsäure-methylester; Smp. 103-104,5°C.

d) 2-[p-[(6-Chlor-2-chinolinyl)oxy]phenoxy]propionsäure und

- 2-Hydroxyäthylcarbaminsäure-methylester den 2-[[2-[p-[(6-Chlor-2-chinolinyl)oxy]phenoxy]propionyl]oxy]-äthylcarbaminsäure-methylester; Smp. 89-90°C.

— 2-Hydroxypropylcarbaminsäure-methylester den 2-[[2-[p-[(6-Chlor-2-chinolinyl)oxy]phenoxy]propionyl]oxy]-propylcarbaminsäure-methylester; NMR (60 MHz, $CDCl_3$) 1,25 ppm (2d, 3p), 1,65 ppm (d, 3p), 3,3 ppm (m, 2p), 3,6 ppm (2s, 3p), 4,8 ppm (q, 1p), 5,01 ppm (q, 1p), 6,7-8,1 ppm (9p).

e) D-2-[p-[(6-Chlor-2-chinolinyl)oxy]phenoxy]propionsäure und

— 2-Hydroxyäthylcarbaminsäure-methylester den 2-[[D-2-[p-[(6-Chlor-2-chinolinyl)oxy]phenoxy]propionyl]oxy]-äthylcarbaminsäure-methylester; Smp. 94-97°C, $[\alpha]_D^{20}$ +30,176° (c = 1,077% in $CHCl_3$).

— 2-Hydroxypropylcarbaminsäure-methylester den 2-[[D-2-[p-[(6-Chlor-2-chinolinyl)oxy]phenoxy]propionyl]oxy]-propylcarbaminsäure-methylester; Smp. 76-78°, $[\alpha]_D^{20}$ + 28,42° (c = 1,05% in $CHCl_3$).

f) D-2-[p-[(6-Brom-2-chinolinyl)oxy]phenoxy]-propionsäure und

— 2-Hydroxyäthylcarbaminsäure-äthylester den 2-[[D-2-[p-[(6-Brom-2-chinolinyl)oxy]phenoxy]propionyl]oxy]-äthylcarbaminsäure-äthylester; Smp. 68-71°C, $[\alpha]_D^{20}$ + 25,57° (c = 0,7% in $CHCl_3$).

g) D-2-[p-[(6-Fluor-2-chinolinyl)oxy]phenoxy]-propionsäure und

— 2-Hydroxyäthylcarbaminsäure-äthylester den 2-[[D-2-[p-[(6-Fluor-2-chinolinyl)oxy]phenoxy]propionyl]oxy]-äthylcarbaminsäure-äthylester; Smp. 95-97°, $[\alpha]_D^{20}$ + 31,003° (c = 1,05% in $CHCl_3$).

— 2-Hydroxyäthylcarbaminsäure-methylester den 2-[[D-2-[p-[(6-Fluor-2-chinolinyl)oxy]phenoxy]propionyl]-oxy]äthylcarbaminsäure-methylester; Smp. 84-86°C, $[\alpha]_D^{20}$ + 31,07° (c = 1,01% in $CHCl_3$).

— 2-Hydroxypropylcarbaminsäure-methylester den 2-[[D-2-[p-[(6-Fluor-2-chinolinyl)oxy]phenoxy]propionyl]oxy]propylcarbaminsäure-methylester; $[\alpha]_D^{20}$ + 26,3° (c = 1,0% in $CHCl_3$), NMR (60 MHz, $CDCl_3$) 1,25 ppm (2d, 3p), 1,67 ppm (d, 3p), 3,32 ppm (m, 2p), 3,65 ppm (2s, 3p), 4,80 ppm (q, 1p), 5,10 ppm (m, 1p), 6,8-8,2 ppm (9p).

h) D-2-[p-[[5-(Trifluormethyl)-2-pyridyl]oxy]phenoxy]-propionsäure und

- 2-Hydroxyäthylcarbaminsäure-methylester den 2-[[D-2-[p-[[5-(Trifluormethyl)-2-pyridyl]oxy]phenoxy]propionyl]-oxy]äthylcarbaminsäure-methylester; $n_D^{20}$ = 1,5022, $[\alpha]_D^{22}$ +31,36° (c = 1,03 in $CHCl_3$);

- 2-Hydroxyäthylcarbaminsäure-äthylester den 2-[[D-2-[p-[[5-(Trifluormethyl)-2-pyridyl]oxy]phenoxy]propionyl]-oxy]äthylcarbaminsäure-äthylester; Smp. 63-64°C, $[\alpha]_D^{22}$ +30,62° (c = 0,96% in $CHCl_3$);

— 2-Hydroxypropylcarbaminsäure-methylester den 2-[[D-2-[p-[[5-(Trifluormethyl)-2-pyridyl]oxy]phenoxy]propionyl]-oxy]propylcarbaminsäure-methylester; $\alpha_D^{20}$ + 24,474° (c = 1,08% in $CHCl_3$); $n_D^{20}$ = 1,5043.

i) D-2-[p-[[5-Difluorchlormethyl)-2-pyridyl]oxy]-phenoxy]propionsäure und

— 2-Hydroxyäthylcarbaminsäure-methylester den 2-[[D-2-[p-[[5-(Difluorchlormethyl)-2-pyridyl]oxy]phenoxy]-propionyl]oxy]äthylcarbaminsäure-methylester; $n_D^{20}$ = 1,5292; $[\alpha]_D^{20}$ +29,133° (c = 0,99% in $CHCl_3$);

— 2-Hydroxyäthylcarbaminsäure-äthylester den 2-[[D-2-[p-[[5-(Difluorchlormethyl)-2-pyridyl]oxy]phenoxy]-propionyl]oxy]äthylcarbaminsäure-äthylester; $n_D^{20}$ = 1,5201; $[\alpha]_D^{20}$ +27,77° (c = 0,998% in $CHCl_3$);

— 2-Hydroxypropylcarbaminsäure-methylester den 2-[[D-2-[p-[[5-(Difluorchlormethyl)-2-pyridyl]oxy]phenoxy]propionyl]-

oxy]propylcarbaminsäure-methylester; $n_D^{20}$ = 1,5127; $[\alpha]_D^{20}$ +24,87° (c = 1,046% in $CHCl_3$).

k) 2-[p-[(5-Difluorchlormethyl)-2-pyridyl]oxy]phenoxy]-propionsäure und

– 2-Hydroxyäthylcarbaminsäure-methylester den 2-[[2-[p-[(5-Difluorchlormethyl)-2-pyridyl]oxy]phenoxy]-propionyl]oxy]äthylcarbaminsäure-methylester; 1H-NMR ($CDCl_3$, 60 MHz) 8,46 ppm (m,1H), 7,93 ppm (dd, 1H), 7,28-6,8 ppm (m, 5H), 5,13-4,73 ppm (m, 1H), 4,83 ppm (q, 1H), 4,30 ppm (t, 2H), 3,67 ppm (s, 3H), 3,57-3,23 ppm (m, 2H), 1,65 ppm (d, 3H).

2-[p-[[5-(Trifluormethyl)-2-pyridyl]oxy]phenoxy]-propionsäure und

– 2-Hydroxyäthylcarbaminsäure-methylester den 2-[[2-[p-[[5-(Trifluormethyl)-2-pyridyl]oxy]phenoxy]propionyl]-oxy]-äthylcarbaminsäure-methylester; Smp. 56-58,5°C;

– 2-Hydroxyäthylcarbaminsäure-äthylester den 2-[[2-[p-[[5-(Trifluormethyl)-2-pyridyl]oxy]phenoxy]propionyl]-oxy]-äthylcarbaminsäure-äthylester; 1H-NMR ($CDCl_3$, 60 MHz) 8,40 ppm (m,1H), 7,86 (dd, 1H), 7,20-6,80 ppm (m, 5H), 4,97-4,57 ppm (m, 1H), 4,80 ppm (q, 1H), 4,43-3,93 ppm (m, 4H), 3,57-3,23 ppm (m, 2H), 1,65 ppm (d, 3H), 1,22 ppm (t, 3H).

– 2-Hydroxypropylcarbaminsäure-methylester den 2-[[2-[p-[[5-(Trifluormethyl)-2-pyridyl]oxy]phenoxy]propionyl]-oxy]-propylcarbaminsäure-methylester; 1H-NMR ($CDCl_3$, 400 MHz) 8,41 ppm (m, 1H), 7,88 ppm (dd, 1H), 7,10-6,90 ppm (m, 5H), 5,10-5,0 ppm (m, 1H), 4,9-4,47 ppm (m, 2H), 3,66 und 3,61 ppm (2s, 3H), 3,46-3,09 ppm (m, 2H), 1,64 und 1,62 ppm (2d, 3H), 1,26 und 1,20 ppm (2d, 3H).

– 1-Aethyl-2-hydroxyäthylcarbaminsäure-äthylester den 2-[[2-[p-[[5-(Trifluormethyl)-2-pyridyl]oxy]phenoxy]propionyl]-oxy]-1-(äthyl)äthylcarbaminsäure-äthylester; 1H-NMR ($CDCl_3$,

400 MHz) 8,42 ppm (m, 1H), 7,88 ppm (dd, 1H), 7,0-6,88 ppm (m, 5H), 4,79 ppm (2q, 1H), 4,64-4,43 ppm (m, 1H), 4,30-4,27 ppm (m, 1H), 4,17-4,01 ppm (m, 3H), 3,85-3,70 ppm (m, 1H), 1,64 und 1,63 ppm (2d, 3H), 1,55-1,30 ppm (m, 2H), 1,29-1,14 ppm (m, 3H), 0,91 und 0,89 ppm (2t, 3H).

- N-(2-Hydroxyäthyl)-N-methylcarbaminsäure-methylester den 2-[[-[p-[[5-Trifluormethyl)-2-pyridyl]oxy]phenoxy]propionyl]-oxy]-N-methyl-äthylcarbaminsäure-methylester; 1H-NMR ($CDCl_3$, 60 MHz) 8,45 ppm (m, 1H), 7,91 ppm (dd, 1H), 7,25-6,8 ppm (m, 5H), 4,79 ppm (q, 1H), 4,32 ppm (t, 2H), 3,72 ppm (s, 3H), 3,55 ppm (t, 2H), 2,90 ppm (s, 3H), 1,62 ppm (d, 3H).

- 2-Hydroxyäthylcarbaminsäure-2-chloräthylester den 2-[[-[p-[[5-(Trifluormethyl)-2-pyridyl]oxy]phenoxy]propionyl]-oxy]-äthylcarbaminsäure-2-chloräthylester; 1H-NMR ($CDCl_3$-60 MHz) 8,45 ppm (m, 1H), 7,92 ppm (dd, 1H), 7,25-6,80 ppm (m, 5H), 5,1-4,61 ppm (m, 2H), 4,5-4,17 ppm (m, 4H), 3,8-3,2 ppm (m, 4H), 1,65 ppm (d, 3H).

- 2-Hydroxypropylcarbaminsäure-isopropylester den 2-[[2-[p-[[5-(Trifluormethyl)-2-pyridyl]oxy]phenoxy]propionyl]-oxy]-propylcarbaminsäure-isopropylester; 1H-NMR ($CDCl_3$, 60 MHz) 8,46 ppm (m, 1H), 7,91 ppm (dd, 1H), 7,24-6,8 ppm (m, 5H), 5,26-4,45 ppm (m, 4H), 3,52-3,06 ppm (m, 2H), 1,63 ppm (d, 3H), 1,22 ppm (d, 9H).

- 2-Hydroxyäthylcarbaminsäure-isobutylester den 2-[[2-[p-[[5-(Trifluormethyl)-2-pyridyl]oxy]phenoxy]propionyl]-oxy]-äthylcarbaminsäure-isobutylester; 1H-NMR ($CDCl_3$, 60 MHz) 8,45 ppm (m, 1H), 7,92 ppm (dd, 1H), 7,25-6,8 ppm (m, 5H), 4,82 ppm (q, 1H), 5,07-4,6 ppm (m, 1H), 4,29 ppm (t, 2H), 3,85 ppm (d, 2H), 3,58-3,23 ppm (m, 2H), 1,90 ppm (m, 1H), 1,65 ppm (d, 3H), 0,90 ppm (d, 6H).

- 2-Hydroxyäthylcarbaminsäure-butylester den 2-[[2-[p-[[5-(Trifluormethyl)-2-pyridyl]oxy]phenoxy]propionyl]-oxy]-äthylcarbaminsäure-butylester; 1H-NMR ($CDCl_3$, 60 MHz)

8,45 ppm (m, 1H), 7,91 ppm (dd, 1H), 7,27-6,78 ppm (m, 5H), 5,03-4,6 ppm (m, 1H), 4,82 ppm (q, 1H), 4,52-3,92 ppm (m, 4H), 3,6-3,22 (m, 2H), 1,9-1,1 ppm (m, 4H), 1,63 ppm (d, 3H), 0,93 (t, 3H).

Der oben als Ausgangsmaterial verwendete 2-Hydroxyäthylcarbaminsäure-isobutylester kann wie folgt hergestellt werden:

42,8 g Aethanolamin werden in 350 ml Methylenchlorid vorgelegt. Dann lässt man 47,8 g Chlorameisensäure-isobutylester während 1 Stunde bei 5-15°C unter Eiskühlung zutropfen. Nach weiteren 4 Stunden Rühren bei Raumtemperatur wird das Methylenchlorid abgedampft und der Rückstand destilliert. Man erhält reinen (2-Hydroxyäthyl)-carbaminsäure-isobutylester; Sdp. 111-113°C/0,07 Torr.

## Beispiel 2

Ein Gemisch von 1,99 g 2,6-Dichlorchinoxalin, 2,83 g 2-[[2-(4-Hydroxyphenoxy)propionyl]oxy]äthylcarbaminsäure-methylester, 1,4 g Kaliumcarbonat und 20 ml N,N-Dimethylformamid wird über Nacht bei Raumtemperatur gerührt. Zur Aufarbeitung wird das Gemisch dann auf Eiswasser gegossen und mit Aethylacetat extrahiert. Die Extrakte werden mit Wasser und Kochsalzlösung gewaschen, über Natriumsulfat getrocknet und eingedampft. Durch Chromatographie an Kieselgel mit Hexan-Aethylacetat (2:1) erhält man reinen 2-[[2-[p-[(6-Chlor-2-chinoxalinyl)oxy]phenoxy]propionyl]-oxy]äthylcarbaminsäure-methylester; Smp. 103-104.5°C.

In analoger Weise erhält man ausgehend von 2-[[D-2-(4-Hydroxyphenoxy)propionyl]oxy]äthylcarbaminsäure-methylester den 2-[[D-2-[p-[(6-Chlor-2-chinoxalinyl)oxy]phenoxy]-propionyl]oxy]äthylcarbaminsäure-methylester; Smp. 93-95°C.

Der oben als Ausgangsmaterial verwendete 2-[[2-(4-Hydroxyphenoxy)propionyl]oxy]äthylcarbaminsäure-methylester kann wie folgt erhalten werden:

9,0 g 2-Hydroxyäthylcarbaminsäure-methylester und 6,7 g Pyridin werden in 70 ml Diäthyläther gelöst und auf 0-5°C gekühlt. Nun werden 18,0 g 2-Brompropionsäurebromid, gelöst in 20 ml Diäthyläther, innerhalb von 30 Minuten zugetropft, und das Gemisch wird noch 30 Minuten bei Eistemperatur und 1 Stunde bei Raumtemperatur gerührt. Zur Aufarbeitung wird das Gemisch dann auf Wasser gegossen und mit Diäthyläther extrahiert. Die Aetherphase wird mit Kochsalzlösung zweimal gewaschen, über wasserfreiem Natriumsulfat getrocknet und unter vermindertem Druck eingedampft. Man erhält 2-(2-Brompropionyloxy)äthylcarbaminsäure-methylester als gelbes Oel, das ohne weitere Reinigung zur Reaktion eingesetzt werden kann.

Ein Gemisch bestehend aus 2,8 g Hydrochinon-monobenzyläther, 3,56 g 2-(2-Brompropionyloxy)äthylcarbaminsäure-methylester, 3,87 g Kaliumcarbonat und 30 ml Aceton wird 2 Stunden unter Rühren am Rückfluss erhitzt. Das abgekühlte Reaktionsgemisch wird filtriert und das Filtrat eingedampft. Der Rückstand gibt nach Flash-Chromatographie reinen 2-[[2-(4-Benzyloxyphenoxy)propionyl]oxy]äthylcarbaminsäure-methylester vom Smp. 79-81°C. Davon wird 1,0 g in 100 ml Essigsäureäthylester gelöst und in Gegenwart von 0,1 g 5% Palladium auf Kohle hydriert. Nach 45 Minuten Schütteln ist die $H_2$-Aufnahme beendet. Der Katalysator wird abfiltriert und das Lösungsmittel am Rotationsverdampfer entfernt. Man erhält den 2-[[2-(4-Hydroxyphenoxy)propionyl]-oxy]äthylcarbaminsäure-methylester; $n_D^{20}$ = 1,5003.

Das für die Herstellung des 2-[[D-2-[p-[(6-Chlor-2-chinoxalinyl)oxy]phenoxy]propionyl]oxy]äthylcarbaminsäure-methylesters erforderliche D-Ausgangsmaterial kann wie folgt erhalten werden:

2,0 g D-2-(4-Hydroxyphenoxy)propionsäure werden mit 13,1 g Thionylchlorid während 1,5 Stunden am Rückfluss erhitzt. Dann wird das überschüssige Thionylchlorid abdestilliert und der Rückstand am Hochvakuum getrocknet. Das so erhaltene Säurechlorid wird mit 20 ml Methylchlorid aufgenommen und mit 1,3 g Pyridin versetzt. Dann tropft man bei 0-5°C während 15 Minuten eine Lösung von 1,31 g 2-Hydroxyäthylcarbaminsäure-methylester in 20 ml Methylenchlorid zu und lässt über Nacht bei Raumtemperatur reagieren. Zur Aufarbeitung wird das Reaktionsgemisch am Rotationsverdampfer eingeengt und dann auf Eiswasser gegossen. Man extrahiert zweimal mit Aethylacetat und wäscht die Extrakte mit Wasser und Kochsalzlösung. Das nach Trocknen über Natriumsulfat und Eindampfen erhaltene Rohprodukt wird durch Chromatographie an Kieselgel gereinigt. Man erhält den 2-[[D-2-(4-Hydroxyphenoxy)propionyl]oxy]äthylcarbaminsäure-methylester; $n_D^{20}$ = 1,5201.

Das oben als Ausgangsmaterial verwendete 2,6-Dichlorchinoxalin kann wie folgt erhalten werden:

1,44 g Natriumhydrid (98%ig) werden in 10 ml Dimethylsulfoxyd vorgelegt. Eine Lösung von 10,4 g 4-Chlor-2-nitroanilin in 40 ml Dimethylsulfoxyd wird so zugetropft, dass die Reaktionstemperatur nicht über 30°C ansteigt. Nach erfolgter Zugabe wird noch 30 Minuten nachgerührt und dann werden 13,25 g Diäthoxyessigsäureäthylester rasch zugegeben. Man rührt über Nacht bei Raumtemperatur, giesst dann auf 150 ml Wasser und extrahiert mit Aethylacetat. Die Extrakte werden mit Wasser nachgewaschen, getrocknet und eingedampft. Der Rückstand wird aus Aethanol/Wasser umkristallisiert. Man erhält 4'-Chlor-2,2-diäthoxy-2'-nitroacetanilid vom Smp. 74-76°C.

In analoger Weise erhält man ausgehend von 4-Fluor-2-nitroanilin das 4'-Fluor-2,2-diäthoxy-2'-nitroacetanilid vom Smp. 78-81°C.

0,8 g Raney-Nickel werden im Hydrierkolben vorgelegt und mit 50 ml Aethanol aufgeschlämmt. Dazu gibt man 7,6 g 4'-Chlor-2,2-diäthoxy-2'-nitroacetanilid und hydriert bei Raumtemperatur und Normaldruck. Nach 2 Stunden ist die Aufnahme von Wasserstoff beendet. Nach Abnutschen und Einengen des Filtrates wird das Produkt mit Wasser ausgefällt. Man erhält 4'-Chlor-2,2-diäthoxy-2'-aminoacetanilid vom Smp. 89-90°C.

In analoger Weise erhält man ausgehend von 4'-Fluor-2,2-diäthoxy-2'-nitroacetanilid das 4'-Fluor-2,2-diäthoxy-2'-aminoacetanilid vom Smp. 51-53°C.

136 g 4'-Chlor-2,2-diäthoxy-2'-aminoacetanilid werden in 2000 ml Aethanol gelöst; dann wird mit 630 ml Wasser verdünnt. Nach Zugabe von 132 g Amberlyst 15 (Fluka 66423) wird 4 Stunden bei Rückflusstemperatur gerührt. Das Reaktionsgemisch wird heiss abfiltriert, um den Amberlyst zu entfernen. Die Reaktionslösung wird auf 0°C abgekühlt und das auskristallisierte Produkt abfiltriert. Die Mutterlauge wird eingeengt, weitere Kristalle wiederum abfiltriert.

Das Produkt, gelb bis beige Kristalle, wird unter vermindertem Druck bei 80°C getrocknet. Das erhaltene 2-Hydroxy-6-chlorchinoxalin schmilzt bei 323°C.

In analoger Weise erhält man aus 4'-Fluor-2,2-diäthoxy-2'-aminoacetatanilid das 2-Hydroxy-6-fluorchinoxalin, Smp. 304-305°C.

45 g 2-Hydroxy-6-chlorchinoxalin werden im Sulfierkolben mit 100 ml Phosphoroxychlorid versetzt. Das Gemisch wird 20 Minuten bei Rückflusstemperatur gerührt. Hierauf wird das überschüssige Phosphoroxychlorid abdestilliert und der Rückstand mit 500 ml Eiswasser versetzt. Das erhaltene Produkt wird abfiltriert und mit Wasser bis zur

Neutralität gewaschen. Durch Kristallisation aus Aethanol/ Wasser erhält man 2,6-Dichlorchinoxalin mit einem Schmelzpunkt von 152°C.

In analoger Weise erhält man aus 2-Hydroxy-6-fluorchinoxalin das 2-Chlor-6-fluor-chinoxalin, Smp. 152°C.

## II. Formulierungsbeispiele

### Beispiel 3

Zur Herstellung eines emulgierbaren Konzentrates werden die nachstehend aufgeführten Bestandteile miteinander vermischt:

Emulgierbares Konzentrat 1

| | |
|---|---|
| Wirkstoff der Formel I | 250 g |
| Ricinusöl-(20)-äthoxylat | 50 g |
| Dodecylbenzolsulfonsäure-Calciumsalz | 25 g |
| Lösungsmittelgemisch aus Aethylbenzolen | 150 g |
| N-Methyl-2-pyrrolidon | auf 1000 ml |

Emulgierbares Konzentrat 2

| | |
|---|---|
| Wirkstoff der Formel I | 125 g |
| Ricinusöl-(20)-äthoxylat | 50 g |
| Dodecylbenzolsulfonsäure-Calciumsalz | 25 g |
| N-Methyl-2-pyrrolidon | auf 1000 ml |

Das so erhaltene Konzentrat (1 oder 2) emulgiert spontan in Wasser, und die sich ergebende Emulsion eignet sich als gebrauchsfertige Spritzbrühe.

### Beispiel 4

Zur Herstellung eines Spritzpulvers werden die nachstehend aufgeführten Bestandteile miteinander vermischt und hierauf feingemahlen:

| | |
|---|---|
| Wirkstoff der Formel I | 25 |
| Hydratisierte Kieselsäure | 5 |
| Alkylphenol-(12)-äthoxylat | 4 |
| Polycarbonsäure-Natriumsalz | 4 |
| Kieselgur | 62 |

Das so erhaltene Pulver kann mit Wasser benetzt werden. Die entstandene Suspension eignet sich als gebrauchsfertige Spritzbrühe.

## Beispiel 5

Zur Herstellung eines Stäubemittels werden die nachstehend aufgeführten Bestandteile miteinander vermischt und hierauf feingemahlen:

| | Gewichtsprozent |
|---|---|
| Wirkstoff der Formel I | 10 |
| Hydratisierte Kieselsäure | 7,5 |
| Talcum | 82,5 |

## Beispiel 6

Zur Herstellung eines Granulats werden die nachstehend aufgeführten Bestandteile miteinander vermischt:

| | Gewichtsprozent |
|---|---|
| Wirkstoff der Formel I | 5 |
| Dipropylenglykol | 5 |
| Bimssteingranulat | 90 |

## Patentansprüche

1. Verbindungen der allgemeinen Formel

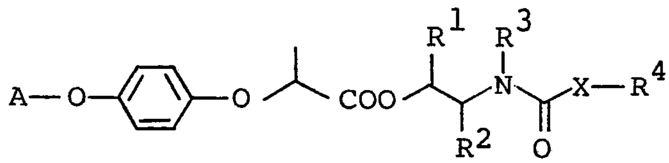

I

worin A eine der Gruppen (a) und (b)

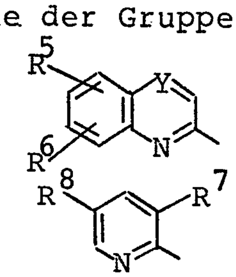

(a)

(b)

darstellt, worin $R^1$ Wasserstoff, Methyl oder Aethyl, $R^2$ Wasserstoff, Methyl oder Aethyl, $R^3$ Wasserstoff oder $C_{1-4}$Alkyl, $R^4$ $C_{1-4}$Alkyl oder halogensubstituiertes $C_{1-4}$Alkyl, $R^5$ Halogen, $C_{1-4}$-Alkyl, halogensubstituiertes Methyl, $C_{1-4}$-Alkoxy, halogensubstituiertes Methoxy, $R^6$ Wasserstoff oder Chlor, $R^7$ Wasserstoff oder Chlor, $R^8$ Chlor, halogensubstituiertes Methyl, X Sauerstoff oder Schwefel und Y Stickstoff oder -CH= bedeuten.

2. Verbindungen nach Anspruch 1, dadurch gekennzeichnet, dass $R^5$ Halogen, $C_{1-4}$-Alkyl, Trifluormethyl, $C_{1-4}$-Alkoxy, Difluormethoxy oder Trifluormethoxy und $R^8$ Chlor oder Trifluormethyl bedeuten.

3. Verbindungen nach Anspruch 1 oder 2, dadurch gekennzeichnet, dass A eine Gruppe der Formel (a) darstellt, worin Y Stickstoff bedeutet.

4. Verbindungen nach Anspruch 1, 2 oder 3, dadurch

gekennzeichnet, dass X Sauerstoff bedeutet.

5. Verbindungen nach einem der Ansprüche 1-4, dadurch gekennzeichnet, dass $R^2$ und $R^3$ Wasserstoff bedeuten.

6. Verbindungen nach einem der Ansprüche 1 bis 5, dadurch gekennzeichnet, dass $R^4$ Methyl oder Aethyl bedeutet.

7. Verbindungen nach einem der Ansprüche 1 bis 6, dadurch gekennzeichnet, dass $R^5$ Fluor, Chlor oder Trifluormethyl bedeutet.

8. Verbindungen nach einem der Ansprüche 1 bis 7, dadurch gekennzeichnet, dass $R^6$ Wasserstoff bedeutet.

9. Verbindungen nach einem der Ansprüche 1 bis 6, dadurch gekennzeichnet, dass $R^8$ Chlor oder Trifluormethyl bedeutet.

10. 2-[[2-[p-[(6-Chlor-2-chinoxalinyl)oxy]phenoxy]-propionyl]oxy]äthylcarbaminsäure-methylester.

11. 2-[[2-[p-[(6-Chlor-2-chinoxalinyl)oxy]phenoxy]-propionyl]oxy]propylcarbaminsäure-methylester.

12. Verbindungen nach einem der Ansprüche 1 bis 11 in D-Form.

13. Eine Verbindung aus der Gruppe 2-[[D-2-[p-[[5-(Trifluormethyl)-2-pyridyl]oxy]phenoxy]propionyl]oxy]-äthylcarbaminsäure-methylester, 2-[[D-2-[p-[[5-(Trifluormethyl)-2-pyridyl]oxy]phenoxy]propionyl]oxy]äthylcarbaminsäure-äthylester, 2-[[D-2-[p-[(6-Chlor-2-chinoxalinyl)-oxy]phenoxy]propionyl]oxy]-N-methyl-äthylcarbaminsäure-methylester, 2-[[D-2-[p-[(6-Chlor-2-chinoxalinyl)oxy]phenoxy]-propionyl]oxy]1-(äthyl)äthylcarbaminsäure-äthylester, 2-[[D-2-[p-[(6-Chlor-2-chinoxalinyl)oxy]phenoxy]propionyl]-oxy]äthylcarbaminsäure-isobutylester,

2-[[D-2-[p-[(6-Fluor-2-chinoxalinyl)oxy]phenoxy]propionyl]-oxy]äthylcarbaminsäure-äthylester und 2-[[D-2-[p-[(6-Fluor-2-chinoxalinyl)oxy]phenoxy]propionyl]-oxy]propylcarbaminsäure-methylester.

14. Verfahren zur Herstellung von Verbindungen der Formel I in Anspruch 1, dadurch gekennzeichnet, dass man

a) eine substituierte Phenoxy-propionsäure der allgemeinen Formel

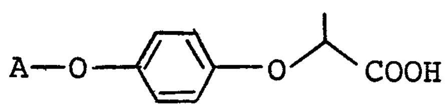

II

worin A die oben angegebene Bedeutung besitzt, oder ein reaktionsfähiges Derivat davon, mit einer Verbindung der allgemeinen Formel

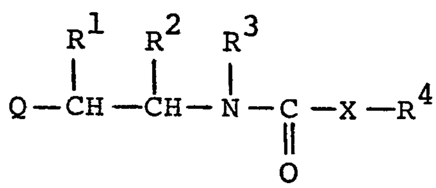

III

worin $R^1$, $R^2$, $R^3$, $R^4$ und X die oben angegebenen Bedeutungen besitzen und Q eine Abgangsgruppe darstellt,

umsetzt, oder

b) eine Verbindung der allgemeinen Formel

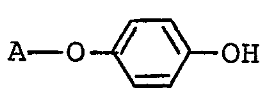

IV

worin A die oben angegebene Bedeutung besitzt, oder ein Alkalimetallsalz davon, mit einer Verbindung der allgemeinen Formel

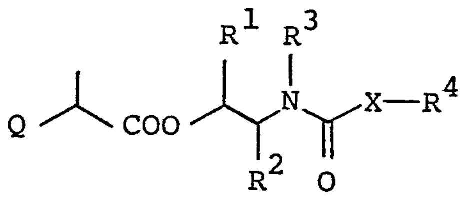

V

worin Q eine Abgangsgruppe darstellt und $R^1$, $R^2$, $R^3$, $R^4$ und X die oben angegebenen Bedeutungen besitzen,

erforderlichenfalls in Gegenwart einer Base, umsetzt, oder

c) eine Verbindung der allgemeinen Formel

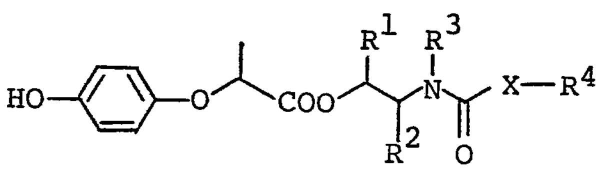

VI

worin $R^1$, $R^2$, $R^3$, $R^4$ und X die oben angegebenen Bedeutungen besitzen,

oder ein Alkalimetallsalz davon, mit einer Verbindung der allgemeinen Formel

AZ VII

worin A eine der obigen Gruppen (a) und (b) darstellt und Z Halogen, insbesondere Chlor, oder Fluor, bedeutet,

umsetzt.

15. Verfahren nach Anspruch 14, dadurch gekennzeichnet, dass man Ausgangsmaterialien verwendet, worin $R^5$ Halogen, $C_{1-4}$-Alkyl, Trifluormethyl, $C_{1-4}$-Alkoxy, Difluormethoxy oder Trifluormethoxy und $R^8$ Chlor oder Trifluormethyl bedeuten.

16. Unkrautbekämpfungsmittel, dadurch gekennzeichnet, dass es eine wirksame Menge einer der Verbindungen gemäss einem der Ansprüche 1-13, sowie Formulierungshilfsstoffe

enthält.

17. Unkrautbekämpfungsmittel nach Anspruch 16, dadurch gekennzeichnet, dass es eine Verbindung enthält, worin $R^5$ Halogen, $C_{1-4}$-Alkyl, Trifluormethyl, $C_{1-4}$-Alkoxy, Difluormethoxy oder Trifluormethoxy und $R^8$ Chlor oder Trifluormethyl bedeuten.

18. Verfahren zur Bekämpfung von Unkräutern, dadurch gekennzeichnet, dass man diese mit einer wirksamen Menge einer der in den Ansprüchen 1 bis 13 genannten Verbindungen bzw. eines in Anspruch 16 genannten Mittels behandelt.

19. Verfahren nach Anspruch 17, dadurch gekennzeichnet, dass man die Unkräuter mit einer Verbindung, worin $R^5$ Halogen, $C_{1-4}$-Alkyl, Trifluormethyl, $C_{1-4}$-Alkoxy, Difluormethoxy oder Trifluormethoxy und $R^8$ Chlor oder Trifluormethyl bedeuten.

20. Verwendung eines der in den Ansprüchen 1 bis 13 genannten Verbindungen bzw. eines in Anspruch 16 genannten Mittels zur Bekämpfung von Unkräutern.

21. Verbindungen der allgemeinen Formel

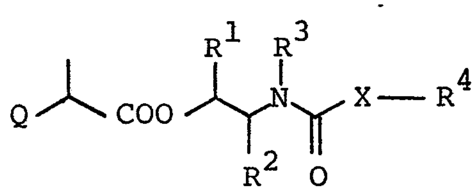

V

worin Q, $R^1$, $R^2$, $R^3$, $R^4$ und X die obigen Bedeutungen besitzen.

22. Verbindungen der allgemeinen Formel

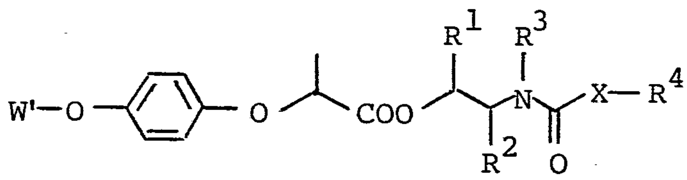

VIIIa

worin $R^1$, $R^2$, $R^3$, $R^4$ und X die obigen Bedeutungen besitzen und W' Wasserstoff oder eine leicht abspaltbare Gruppe darstellt.

23. Verfahren zur Herstellung von 2-Hydroxychinoxalinen der Formel

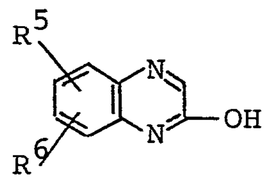

IX

worin $R^5$ und $R^6$ die obige Bedeutung haben, dadurch gekennzeichnet, dass man eine Verbindung der Formel

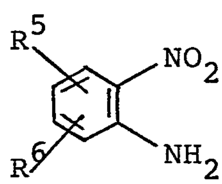

X

mit einem den Rest der Formel

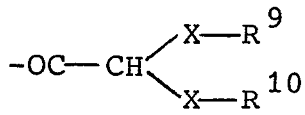

XI

worin $R^9$ und $R^{10}$ die in der Beschreibung angegebenen Bedeutungen haben,

abgebenden Verbindung zu einem Amid der Formel

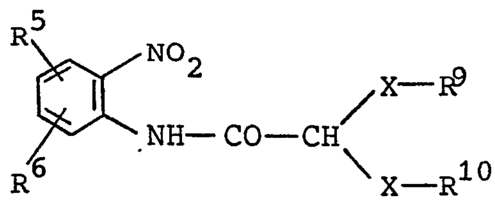

XII

umsetzt, und dass man in beliebiger Reihenfolge die Nitrogruppe in der Verbindung der Formel XII zur Aminogruppe reduziert und die Gruppen $-X-R^9$ und $-X-R^{10}$ abspaltet, wodurch sich unter Ringschluss die Verbindung der Formel IX bildet.

***

<u>Patentansprüche für Oesterreich</u>

1. Unkrautbekämpfungsmittel, dadurch gekennzeichnet, dass es eine wirksame Menge einer Verbindung der allgemeinen Formel

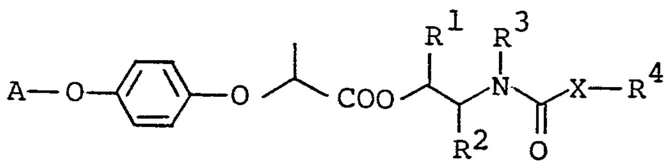

I

worin A eine der Gruppen (a) und (b)

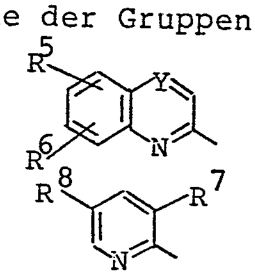

(a)

(b)

darstellt, worin $R^1$ Wasserstoff, Methyl oder Aethyl, $R^2$ Wasserstoff,Methyl oder Aethyl, $R^3$ Wasserstoff oder $C_{1-4}$Alkyl, $R^4$ $C_{1-4}$Alkyl oder halogensubstituiertes $C_{1-4}$Alkyl, $R^5$ Halogen, $C_{1-4}$-Alkyl, halogensubstituiertes Methyl, $C_{1-4}$-Alkoxy, halogensubstituiertes Methoxy, $R^6$ Wasserstoff oder Chlor, $R^7$ Wasserstoff oder Chlor, $R^8$ Chlor, halogensubstituiertes Methyl, X Sauerstoff oder Schwefel und Y Stickstoff oder -CH= bedeuten ,
sowie Formulierungshilfsstoffe enthält.

2. Unkrautbekämpfungsmittel nach Anspruch 1, dadurch gekennzeichnet, dass es eine Verbindung der Formel I enthält, worin $R^5$ Halogen, $C_{1-4}$-Alkyl, Trifluormethyl, $C_{1-4}$-Alkoxy, Difluormethoxy oder Trifluormethoxy und $R^8$ Chlor oder Trifluormethyl bedeuten.

3. Unkrautbekämpfungsmittel nach Anspruch 1 oder 2, dadurch gekennzeichnet, dass es eine Verbindung der Formel I enthält, worin A eine Gruppe der Formel (a) darstellt, worin

Y Stickstoff bedeutet.

4. Unkrautbekämpfungsmittel nach Anspruch 1, 2 oder 3, dadurch gekennzeichnet, dass es eine Verbindung der Formel I enthält, worin X Sauerstoff bedeutet.

5. Unkrautbekämpfungsmittel nach einem der Ansprüche 1-4, dadurch gekennzeichnet, dass es eine Verbindung der Formel I enthält, worin $R^2$ und $R^3$ Wasserstoff bedeuten.

6. Unkrautbekämpfungsmittel nach einem der Ansprüche 1-5, dadurch gekennzeichnet, dass es eine Verbindung der Formel I enthält, worin $R^4$ Methyl oder Aethyl bedeutet.

7. Unkrautbekämpfungsmittel nach einem der Ansprüche 1-6, dadurch gekennzeichnet, dass es eine Verbindung der Formel I enthält, worin $R^5$ Fluor, Chlor oder Trifluormethyl bedeutet.

8. Unkrautbekämpfungsmittel nach einem der Ansprüche 1-7, dadurch gekennzeichnet, dass es eine Verbindung der Formel I enthält, worin $R^6$ Wasserstoff bedeutet.

9. Unkrautbekämpfungsmittel nach einem der Ansprüche 1-8, dadurch gekennzeichnet, dass es eine Verbindung der Formel I enthält, worin $R^8$ Chlor oder Trifluormethyl bedeutet.

10. Unkrautbekämpfungsmittel nach Anspruch 1 oder 2, dadurch gekennzeichnet, dass es den 2-[[2-[p-[(6-Chlor-2-chinoxalinyl)oxy]phenoxy]propionyl]oxy]äthylcarbaminsäuremethylester enthält.

11. Unkrautbekämpfungsmittel nach Anspruch 1 oder 2, dadurch gekennzeichnet, dass es den 2-[[2-[p-[(6-Chlor-2-chinoxalinyl)oxy]phenoxy]propionyl]oxy]propylcarbaminsäuremethylester enthält.

12. Unkrautbekämpfungsmittel nach einem der Ansprüche 1-11, dass es die Verbindung der Formel I in der D-Form enthält.

13. Unkrautbekämpfungsmittel, dadurch gekennzeichnet, dass es eine Verbindung aus der Gruppe 2-[[D-2-[p-[[5-(Trifluormethyl)-2-pyridyl]oxy]phenoxy]propionyl]oxy]-äthylcarbaminsäure-methylester, 2-[[D-2-[p-[[5-(Trifluormethyl)-2-pyridyl]oxy]phenoxy]propionyl]oxy]äthylcarbaminsäure-äthylester, 2-[[D-2-[p-[(6-Chlor-2-chinoxalinyl)-oxy]phenoxy]propionyl]oxy]-N-methyl-äthylcarbaminsäure-methylester, 2-[[D-2-[p-[(6-Chlor-2-chinoxalinyl)oxy]phenoxy]-propionyl]oxy]1-(äthyl)äthylcarbaminsäure-äthylester, 2-[[D-2-[p-[(6-Chlor-2-chinoxalinyl)oxy]phenoxy]propionyl]-oxy]äthylcarbaminsäure-isobutylester, 2-[[D-2-[p-[(6-Fluor-2-chinoxalinyl)oxy]phenoxy]propionyl]-oxy]äthylcarbaminsäure-äthylester und 2-[[D-2-[p-[(6-Fluor-2-chinoxalinyl)oxy]phenoxy]propionyl]-oxy]propylcarbaminsäure-methylester enthält.

14. Verfahren zur Herstellung von Verbindungen der in Anspruch 1 angegebenen Formel I, dadurch gekennzeichnet, dass man

a) eine substituierte Phenoxy-propionsäure der allgemeinen Formel

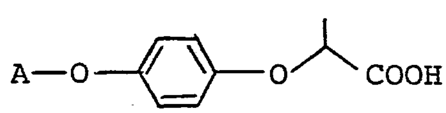

II

worin A die oben angegebene Bedeutung besitzt, oder ein reaktionsfähiges Derivat davon, mit einer Verbindung der allgemeinen Formel

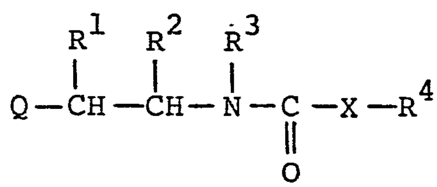

III

worin $R^1$, $R^2$, $R^3$, $R^4$ und X die oben angegebenen Bedeutungen besitzen und Q eine Abgangsgruppe darstellt,

umsetzt, oder

b) eine Verbindung der allgemeinen Formel

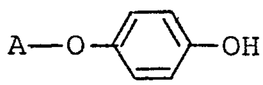

IV

worin A die oben angegebene Bedeutung besitzt,

oder ein Alkalimetallsalz davon, mit einer Verbindung der allgemeinen Formel

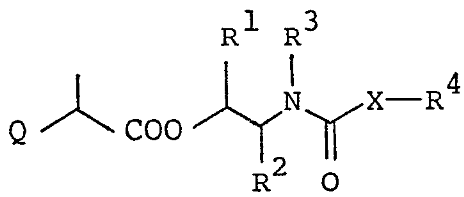

V

worin Q eine Abgangsgruppe darstellt und $R^1$, $R^2$, $R^3$, $R^4$ und X die oben angegebenen Bedeutungen besitzen,

erforderlichenfalls in Gegenwart einer Base, umsetzt, oder

c) eine Verbindung der allgemeinen Formel

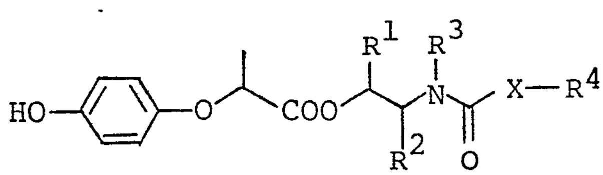

VI

worin $R^1$, $R^2$, $R^3$, $R^4$ und X die oben angegebenen Bedeutungen besitzen,

oder ein Alkalimetallsalz davon, mit einer Verbindung der allgemeinen Formel

AZ VII

worin A eine der obigen Gruppen (a) und (b) darstellt und Z Halogen, insbesondere Chlor, oder Fluor, bedeutet,

umsetzt.

15. Verfahren nach Anspruch 14, dadurch gekennzeichnet, dass man Ausgangsmaterialien verwendet, worin $R^5$ Halogen, $C_{1-4}$-Alkyl, Trifluormethyl, $C_{1-4}$-Alkoxy, Difluormethoxy oder Trifluormethoxy und $R^8$ Chlor oder Trifluormethyl bedeuten.

16. Verfahren zur Bekämpfung von Unkräutern, dadurch gekennzeichnet, dass man diese mit einer wirksamen Menge einer der in Anspruch 1 genannten Verbindungen bzw. eines Mittels gemäss Anspruch 1 behandelt.

17. Verfahren zur Bekämpfung von Unkräutern, dadurch gekennzeichnet, dass man diese mit einer wirksamen Menge einer der in den Ansprüchen 2-13 genannten Verbindungen bzw. eines Mittels gemäss einem der Ansprüche 2-13 behandelt.

18. Verfahren zur Herstellung von 2-Hydroxychinoxalinen der Formel

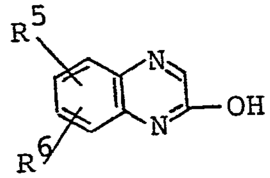

IX

worin $R^5$ und $R^6$ die in Anspruch 1 angegebene Bedeutung haben,

dadurch gekennzeichnet, dass man eine Verbindung der Formel

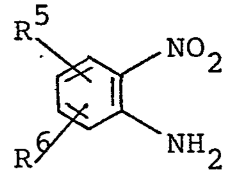

X

mit einem den Rest der Formel

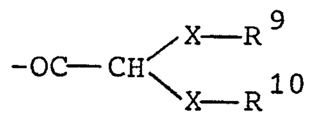

XI

worin $R^9$ und $R^{10}$ die in der Beschreibung angegebenen Bedeutungen haben,

abgebenden Verbindung zu einem Amid der Formel

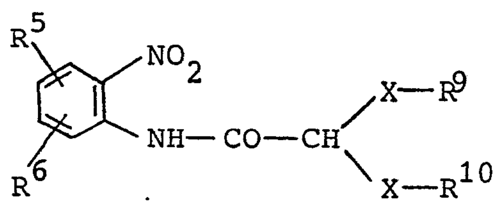

XII

umsetzt, und dass man in beliebiger Reihenfolge die Nitrogruppe in der Verbindung der Formel XII zur Aminogruppe reduziert und die Gruppen $-X-R^9$ und $-X-R^{10}$ abspaltet, wodurch sich unter Ringschluss die Verbindung der Formel IX bildet.

19. Verfahren nach Anspruch 17, dadurch gekennzeichnet, dass $R^5$ in den Formeln IX, X und XII Halogen, $C_{1-4}$-Alkyl, Trifluormethyl, $C_{1-4}$-Alkoxy, Difluormethoxy oder Trifluormethoxy bedeutet.

***